# EUROPEAN PATENT SPECIFICATION

(11) **EP 4 626 897 B1**
(45) Date of publication and mention of the grant of the patent: **08.07.2026**
(21) Application number: 23920194.0
(22) Date of filing: 27.02.2023
(51) Int. Cl.: C07F 1/00, A61K 31/555, A61K 31/513, A61K 31/444, A61P 35/00, A61K 33/34, C07D 213/22, C07D 239/553

(54) **A NEW COPPER(II) COMPLEX CONTAINING 5-FLUOROURACIL AND TERPYRIDINE WITH ANTICANCER AND ANTIANGIOGENIC EFFECTS**
KUPFER(II)-KOMPLEX MIT 5-FLUORURACIL UND TERPYRIDIN MIT ANTIKREBS- UND ANTIANGIOGENER WIRKUNG
NOUVEAU COMPLEXE DE CUIVRE(II) CONTENANT DU 5-FLUOROURACILE ET DE LA TERPYRIDINE PRÉSANTANT DES EFFETS ANTI-CANCEREUX ET ANTI-ANGIOGÉNIQUES

(30) Priority: 02.02.2023 TR 2023001257
(43) Date of publication of application: 08.10.2025
(73) Proprietor: Istinye Üniversitesi, 34010 Zeytinburnu, Istanbul (TR); Karadeniz Teknik Universitesi Teknoloji Transferi Uygulama ve Arastirma Merkezi, 61080 Trabzon (TR)
(72) Inventor: ULUKAYA, Engin, 34010 Zeytinburnu/Istanbul (TR); SARIAL, Zeynep, 34010 Zeytinburnu/Istanbul (TR); AKAR, Remzi Okan, 34010 Zeytinburnu/Istanbul (TR); YILMAZ, Veysel Turan, 61080 Ortahisar/Trabzon (TR); ICSEL YILMAZ, Ceyda, 61080 Ortahisar/Trabzon (TR)
(74) Representative: Yalçiner Patent and Consulting Ltd.
(86) International application number: PCT/TR2023/050190
(87) International publication number: WO 2024/162910

(56) References cited:
- ALPER PINAR ET AL: "Synthesis, characterization, anticancer and antioxidant activity of new nickel(II) and copper(II) flavonoid complexes", JOURNAL OF MOLECULAR STRUCTURE, ELSEVIER AMSTERDAM, NL, vol. 1196, 5 July 2019 (2019-07-05), pages 783 - 792, XP085769210, ISSN: 0022-2860, [retrieved on 20190705], DOI: 10.1016/J.MOLSTRUC.2019.07.009
- ICSEL CEYDA, YILMAZ VEYSEL T., AYGUN MUHITTIN, ERKISA MERVE, ULUKAYA ENGIN: "Novel 5-fluorouracil complexes of Zn(II) with pyridine-based ligands as potential anticancer agents", DALTON TRANSACTIONS, RSC - ROYAL SOCIETY OF CHEMISTRY, CAMBRIDGE, vol. 51, no. 13, 29 March 2022 (2022-03-29), Cambridge , pages 5208 - 5217, XP093199365, ISSN: 1477-9226, DOI: 10.1039/D1DT04070G
- KARGES JOHANNES; XIONG KAI; BLACQUE OLIVIER; CHAO HUI; GASSER GILLES: "Highly cytotoxic copper(II) terpyridine complexes as anticancer drug candidates", INORGANICA CHIMICA ACTA, ELSEVIER BV, NL, vol. 516, 13 November 2020 (2020-11-13), NL , XP086428021, ISSN: 0020-1693, DOI: 10.1016/j.ica.2020.120137
- MOLINARO CAROLINE, MARTORIATI ALAIN, PELINSKI LYDIE, CAILLIAU KATIA: "Copper Complexes as Anticancer Agents Targeting Topoisomerases I and II", CANCERS, MDPI AG, CH, vol. 12, no. 10, CH , pages 2863, XP093199370, ISSN: 2072-6694, DOI: 10.3390/cancers12102863

## Description

### Technical Field of the Invention

The present invention is in the field of chemistry and medicine and relates to the synthesis, anticancer and antiangiogenic effects of a water-soluble 5-fluorouracil (5-FU) copper(II) complex/compound with 2,2':6',2"-terpyridine (terpy), [CuCl(5-FU)(terpy)].

### State of the Art (Prior Art)

Cancer is a disease based on the uncontrolled proliferation of cells in an organ or tissue. According to 2020 data, the estimated number of new cases of cancer in the world is approximately 20 million. It has been reported that 13 million people will die from cancer in 2030, and an increase of 60% is expected in the next 15 years.

Considering all cancer types, it is known that the third most common type of cancer in the world is colorectal cancer. Colorectal cancer has become the predominant cancer, accounting for approximately 10% of cancer-related deaths.

5-Fluorouracil (5-fluoro-1,3-diazinan-2,4-dione, 5-FU) is a fluoropyrimidine analog of uracil fluorinated at the C5 position. 5-FU is an anticancer drug and is widely used, especially for the treatment of colorectal cancer. Its most common use is for colon cancer. 5-FU is incorporated into DNA and RNA and inhibits the action of thymidylate synthase, leading to cancer cell death. Even if 5-FU provides some degree of successful chemotherapy, treatment failure due to drug resistance is still reported in many patients.

5-FU can be considered as a versatile ligand against metal ions. Mixed ligand metal complexes of 5-FU have been shown to exhibit anticancer potential in in vitro studies, on the other hand, studies have shown that 5-FU metal complexes greatly increase cytotoxic activity against various cancer cell lines compared to 5-FU alone.

Ceyda Icsel et al discloses a series of novel Zn(II) complexes of 5-fluorouracilate (5-FU), namely [Zn(5-FU)₂(bpy)] (1), [Zn(5-FU)₂(phen)] (2), [Zn(5-FU)₂(dpya)]·H₂O (3), [Zn(5-FU)₂(bpyma)]·2H₂O (4) and [Zn(5-FU)₂(terpy)]·H₂O (5), synthesized and structurally characterized by spectroscopic methods and X-ray crystallography in "Novel 5-fluorouracil complexes of Zn(ii) with pyridine-based ligands as potential anticancer agents" document. The anti proliferative activity of the complexes was studied against lung (A549), breast (MDA-MB-231), colon (HCT116) and prostate (DU145) cancer cell lines.

In a patent document numbered WO2014003541A1, the invention relates to copper(II)-mixed ligand complexes with anticancer properties, more particularly to copper(II)-polypyridyl complexes with anticancer properties by generation of reactive oxygen species (ROS), proteasome targeting, topoisomerase inhibition and DNA damage.

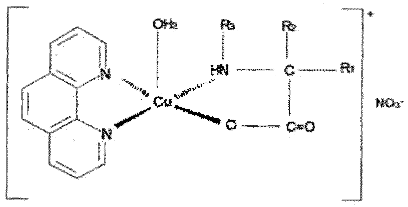

In another patent document numbered EP2257558A1, the invention relates to organocopper complexes and pharmaceutical compositions. It has been reported that they can be used for the treatment of diseases caused by hyperproliferative cells (e.g. cancer cells). It has been reported that copper organocomplexes are synthesized from the reaction of copper(II) acetate with 4-[bis(2-chloroethyl)amino]-D, L-phenylalanine (sarcolysin), N-(2-furanidyl)-5-fluorouracil (tegafur) and aminocarbonylaziridine (leacadin) at acidic or neutral pH in a chloroform/methanol mixture.

P 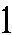 nar Alper et al. discloses new mixed ligand complexes of Ni(II) and Cu(II) synthesized by using flavonoid (quercetin or naringenin) and heterocyclic imine (2,20 :60 ,200-terpyridine or 2,2'-bipyiridine) ligands and their use in cancer treatment in "Synthesis, characterization, anticancer and antioxidant activity of new nickel(II) and copper(II) flavonoid complexes" document. The new complexes are [Ni(narH-1)(terpy)Cl].4H₂O (1, nar ¼ naringenin, terpy ¼ 2,20 :60 ,200-terpyridine), [Cu(narH1)(terpy)Cl].H₂O (2), and [Cu(queH-1)(bpy)(O₃N)].1.5H₂O (3, que ¼ quercetin, bpy ¼ 2,2'-bipyiridine).Antiproliferative effect of complexes were tested by SRB and ATP assays on MCF-7 (breast cancer), A549 (nonsmall cell lung cancer), PC-3 (prostate cancer) and HeLa (human cervical cancer) cell lines.

J. Karges et al. represents highly cytotoxic copper(II) terpyridine complexes as anticancer drug candidates. The biological activity of terminally functionalised mono- and bis-coordinated Cu(II)-2,2':6',2"-terpyridine complexes have been investigated. The bis-coordinated compounds were found to have a cytotoxic effect in the nanomolar range in human adenocarcinomic alveolar basal epithelial cells

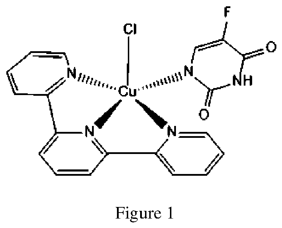

However, there is a need for new copper complexes that can show high efficacy in the treatment of many types of cancer. Figure 1 illustrates the molecular structure of [CuCl(5-FU)(terpy)].

### Brief Description and Objects of the Invention

The primary object of the present invention is to obtain a new [CuCl(5-FU)(terpy)] complex, which is copper(II) containing 5-fluorouracil and terpyridine, which is effective in cancer treatment, especially colon cancer.

In the in-vitro preliminary studies, the newly synthesized [CuCl(5-FU)(terpy)] complex was found to have a high cytotoxic effect on the colon cancer cell line (HCT116) compared to lung cancer (A549), breast cancer (MDA-MB-231), and prostate cancer (DU145) cell lines. In in-vitro studies with [CuCl(5-FU)(terpy)], the complex was also found to have a high cytotoxic effect in other colon cancer cell lines (HCT15, HT29) compared to 5-FU. In addition, in the in-ovo studies with [CuCl(5-FU)(terpy)], the molecule exhibited an antiangiogenic effect (an effect that disrupts tumor vasculature).

The cytotoxicity / cell killing effect with the [CuCl(5-FU)(terpy)] complex was determined by three methods. 1. Sulforhodamine B viability assay, 2. Dual fluorescent staining of morphology with Hoechst 33342 and Propidium Iodide (PI), 3. Determination of cell death mode by flow cytometry method. Consequently, it was found that said new compound showed cytotoxic effect against colon cancer cells in a time- and dose-dependent manner.

### Description of the Figures Illustrating the Invention

The figures and related descriptions prepared for illustrating the effects of the compound of the present invention in colon cancer cell lines are provided below.
**Figure 1****:** Molecular structure of [CuCl(5-FU)(terpy)].
**Figure 2****:** Effects of [CuCl(5-FU)(terpy)] complex and 5-FU at different doses in colon cancer cell lines (HCT15, HCT116, HT29) and healthy cell line (BEAS-2B). NC denotes negative control for cytotoxicity (100 % viability).
**Figure 3****:** Hoechst dye / PI staining at 12, 24 and 48 hours at 100 µM, which is the maximum **dose for** [CuCl(5-FU)(terpy)] in HT29 colon cancer cell line. C denotes control.
**Figure 4****:** Wound Healing (Scratch) Assay with IC₅₀ and GI₅₀ doses of the complex in HT29 cell line.
**Figure 5****:** Effect of [CuCl(5-FU)(terpy)] complex and 5-FU on phosphatidylserine translocation (a marker for apoptosis) in HT29 cell line. Annexin V-FITC test by flow cytometry.
**Figure 6****: Effect of** [CuCl(5-FU)(terpy)] complex and 5-FU on caspase activation (a marker for **apoptosis) in HT29** cell line. Caspase 3/7 activation test by flow cytometry.
**Figure 7****: Effect of** [CuCl(5-FU)(terpy)] complex and 5-FU on DNA in HT29 cell line. DNA Damage (SER139) test by flow cytometry.
**Figure 8****:** Effect of [CuCl(5-FU)(terpy)] complex and 5-FU on oxidative status in HT29 cell line. Oxidative Stress test by flow cytometry.
**Figure 9****:** Antiangiogenic effect of [CuCl(5-FU)(terpy)] at the doses of IC50 and GI50 by employing in-ovo Chorioallantoic Membrane (CAM) assay.

### Detailed Description of the Invention

The present invention relates to the synthesis of a water-soluble 5-fluorouracil (5-FU) copper(II) terpyridine (terpy) complex [CuCl(5-FU)(terpy)] and its use in the treatment of colon cancer.

The cytotoxic effects of this complex on three human colon cancer cell lines (HCT15, HCT116, HT29) were investigated. Effects of the compound on cell viability were investigated by SRB viability assay. The mode of cell death was determined by performing Hoechst dye / PI staining at the relevant doses of the compound. In order to determine the mechanism of cell death, some assays (Annexin V-FITC, Caspase 3/7, DNA Damage (Ser139), and Oxidative Stress tests) were performed by flow cytometry. In addition to these studies, the antiangiogenic effect of the compound was investigated in in-ovo studies.

Synthesis of [CuCl(5-Fu)(terpy)] consists of the following steps;
a. Preparing an aqueous solution of Na(5-FU)·H₂O (0,50 mmol) in 5 mL water,
b. Preparing an aqueous solution of CuCl₂ (0.25 mmol) in 5 mL water,
c. Preparing a solution of terpy (0.25 mmol) in 10 mL methanol,
d. Adding the solution in step "a" to the solution prepared in step "b" with stirring,
e. Adding the solution in step "c" into the mixture,
f. Stirring and filtering the solution at 50-65 °C for 2-4 hours,
g. Filtering and air drying the crystals of the complex.
Elemental analysis composition of [CuCl(5-Fu)(terpy)]: Cu: 13,77%, C: 49,47%, H:2,84%, Cl: 7,68, N: 15,18%. The complex is soluble in water.

### Spectral data of the complex of the invention:

IR (ν/cm⁻¹): 3202vw, 3112m, 3054w, 3016m, 2761w, 1684s, 1645vs, 1616s, 1601s, 1474m, 1450m, 1387w, 1323m, 1292w, 1255vs, 1209s, 1158m, 1039w, 1018m, 1000m, 941w, 900w, 826s, 770vs, 732m, 650m, 644m, 590m, 510m.
UV-vis (H₂O) λₘₐₖₛ/nm (εₘₐₖₛ/M⁻¹cm⁻¹): 265 (64132), 274 (63864), 284 (60867), 324 (38500), 339 (35959), 652 (169), 734 (145).
ESI-MS (m/z, MeOH): 331,6 (42%, calc. 331,0) [CuCl(terpy)]⁺, 295,3 (15%, calc. 295,0) [Cu(terpy) -H)]⁺, 191,2 (100%, calc. 191,9) [Cu(5FU)]⁺.
µₑₜₖ. = 1,67 BM (room temperature).
Molar conductivity, AM (H₂O, 298 K, 1 mM) 12 S cm² mol⁻¹.
Single crystal data: Crystal system: Monoclinic (*P*2₁/n), *a* = 11,6071(9) Å, *b* = 13,0317(14) Å, *c* = 12,3107(11) Å, *α =* 90°, *β* = 91,472(8)°, *y =* 90°. Z = 4.

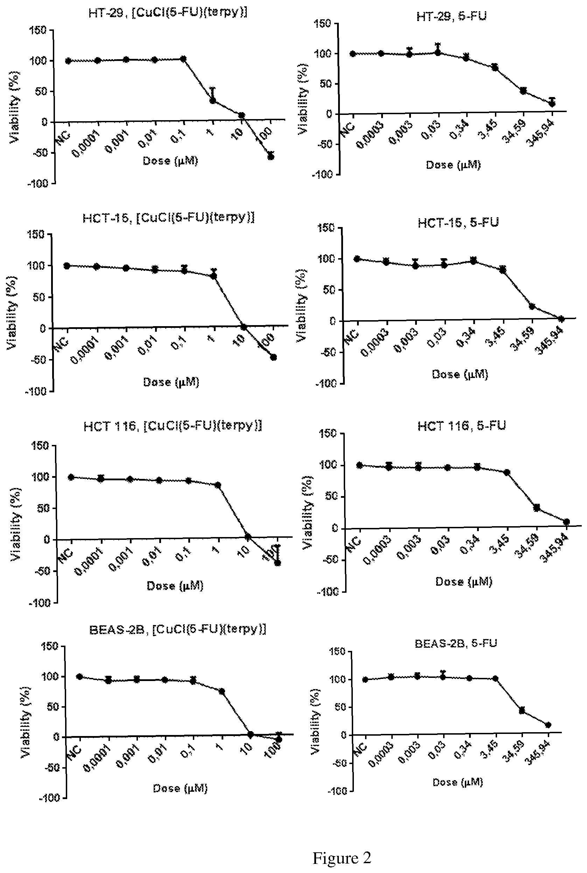

The effect of [CuCl(5-FU)(terpy)] complex and 5-FU on the growth rate of cells was determined by the Sulforhodamine B (SRB) assay (Figure 2). SRB is a bright pink-purple colored water-soluble dye, and it is also used by the American Cancer Institute for the determination of drug-induced cytotoxicity and cell proliferation. The principle of the assay is based on the electrostatic and pH-dependent binding of SRB, a protein dye, to amino acid residues in cells fixed with acetic acid, and the color intensity of the solvent and dye is measured colorimetrically.

The [CuCl(5-FU)(terpy)] complex was administered to colon cancer cell lines (HCT15, HCT116, HT29) and a healthy cell line (BEAS-2B) in ten-fold dilutions starting from 100 µM. And then 48 hours later, cytotoxicity was measured by SRB assay. In a similar manner, 5-FU was administered to colon cancer cell lines (HCT15, HCT116, HT29) and healthy cell line BEAS-2B cells in ten-fold dilutions starting at the suprapharmacological dose of 345.94 µM in human plasma. And then 48 hours later, cytotoxicity was measured by SRB assay. The viability values (% viabilities) obtained by the SRB assay are shown on growth curve in Figure 2. Taking the corresponding doses into account, antigrowth effect of [CuCl(5-FU)(terpy)] complex has been shown to be much higher in colon cancer cell lines (HCT15, HCT116, HT29) compared to 5-FU alone.

Based on these results, the inhibitory concentrations (IC₅₀) at which 50% of cells was viable at 48 hours and the dose that inhibited cell proliferation 50% (GI₅₀) were calculated. When cell lines were compared with each other, both GI₅₀ and IC₅₀ doses were found to be the lowest in HT29 cells. Therefore, it was decided to perform further experiments with HT29 cells only. IC₅₀ = 4.52 µM and GI₅₀ = 1 \µM for HT29 cells.

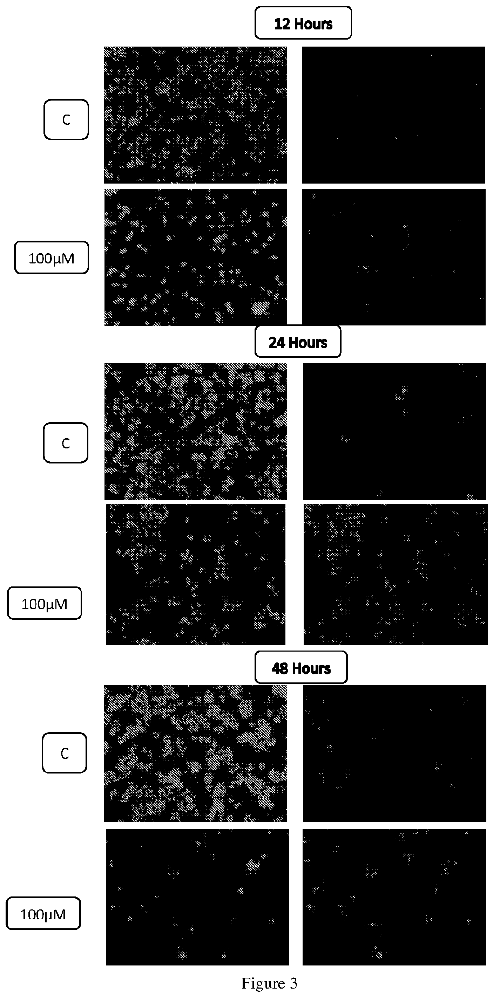

Hoechst dye 33342 and PI dyes are fluorescent dyes used to stain DNA in fluorescence microscopy. One of the methods used in the detection of cell death mode is the dual staining method in which these two dyes are used together. Hoechst dye can stain both living and dead cells. Hoechst dye emits blue/cyan colored fluorescent light. Therefore, the dots marked in blue in Figure 3 show DNA. The other dye used in dual staining is PI, which can only stain DNA of dead (primary or secondary necrotic) cells and glows red. While viable cells with intact membranes are not stained with PI dye. PI dye only stains membrane-damaged (necrotic/dead) cells.

In the present invention, Hoechst dye 33342 / PI staining was performed in HT29 cells at 12, 24 and 48 hours with a maximum dose of 100 µM for [CuCl(5-FU)(terpy)]. In Figure 3, those shown in blue represent both dead and live cells, while those in red represent only dead cells. Blue and red areas (left and right parts of the figure 3) are taken from the same microscopic field. By considering 12, 24, and 48 h Hoechst dye-stained images of HT29 cells, it is observed that the cell population treated with 100 µM [CuCl(5-FU)(terpy)] is much less, compared to the control group (C). In addition, it is observed that the dead cell population (those in red) in the 100µM-treated sample was much higher than in the control group. As a result, a decrease in the HT29 live cell population and an increase in the dead cell population are clearly observed depending on the treatment time. Since the number of dead cells at 48 hours was excessively high, flow cytometry experiments were performed at earlier time points (12 and 24 hours).

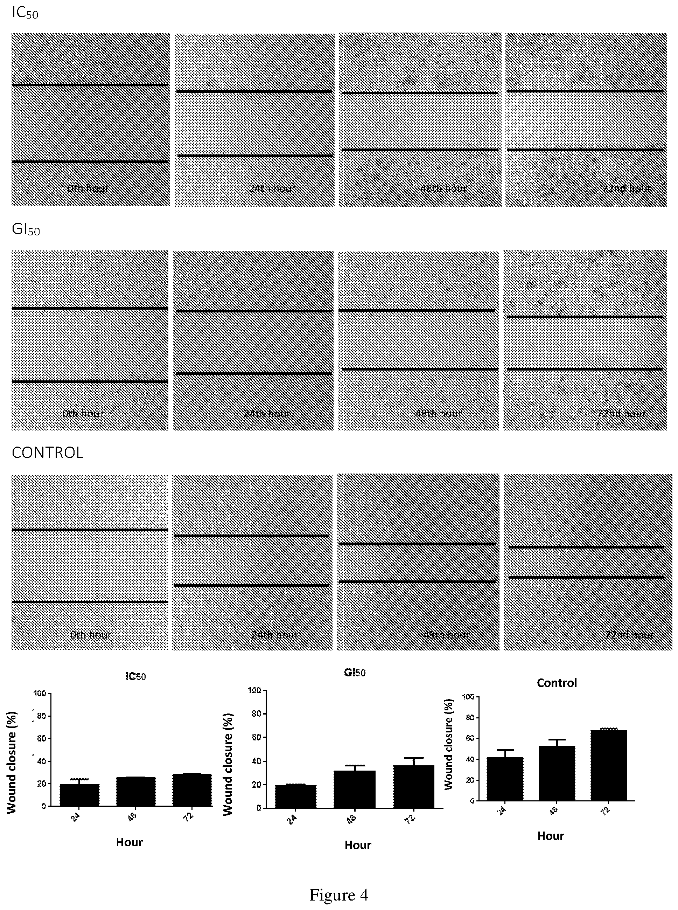

Wound Healing Assay/Scratch Assay is an in vitro laboratory technique used to study cell migration. In the above-mentioned technique, a cavity is created and the capacity (migration) of cells to fill that cavity is measured. Because the complex-treated cells are found to be unable to cover the cavity, it is reported that the complex had antimigratory properties, suggesting an anticancer potential (Figure 4).

In the present invention, when HT29 cells completely cover the bottom of the cell culture dish, a cavity (line) is created with the help of a micropipette tip. Here, the migration of cells, not cell death, is investigated, so there are three experimental groups; IC₅₀ = 4.52 µM, GI₅₀ = 1µM and untreated control group. When the lines are drawn for the experimental groups, this is counted as the 0th hour and the wells are photographed. When the 24th, 48th and 72nd hour photos are taken, the area of the line in all photographs taken at 0, 24, 48 and 72 hours was calculated with the 'ImageJ' program. Area calculations were calculated as Wound Closure(%) and expressed with a graph.

Considering the results, it was observed that there was a significant decrease in the migration of cells between the 48th hour and the 72nd hour at the IC₅₀ dose. Similarly, a decrease in the migration of cells was observed between the 48th hour and the 72nd hour at the GI₅₀ dose. When the control group was examined, it was observed that the migration of cells took place in a healthy manner depending on time.

In the present invention, the mode of cell death (apoptosis) was investigated by flow cytometry using the Annexin V-FITC kit. For the HT29 cell line, 100 µM for [CuCl(5-FU)(terpy)] and 173 µM for 5-FU, which corresponds to the approximate human plasma dose, were used and measurements were performed at 12 and 24 hours.

Phosphotidylserine (PS) is a phospholipid and a component of the cell membrane. Under normal conditions (in untreated cells), it is present in the inner part (the cytoplasmic side) of the cell membrane. However, in the apoptotic cells, they are translocated to outer side of the plasma membrane. Therefore, phosphatidylserine is a marker of apoptotic cells. Annexins consist of a group of proteins that bind to phosphatidylserine in the presence of Ca²⁺.

This test principle uses FITC-labeled Annexin-V to bind to phosphatidylserine. Thus, in the case of apoptotic cells, Annexin V-FITC binds to phosphatidylserine and gives fluorescent signal. In this experiment, PI is also used. PI provides an estimation of the late-stage apoptotic cell (secondary necrotic cell) population.

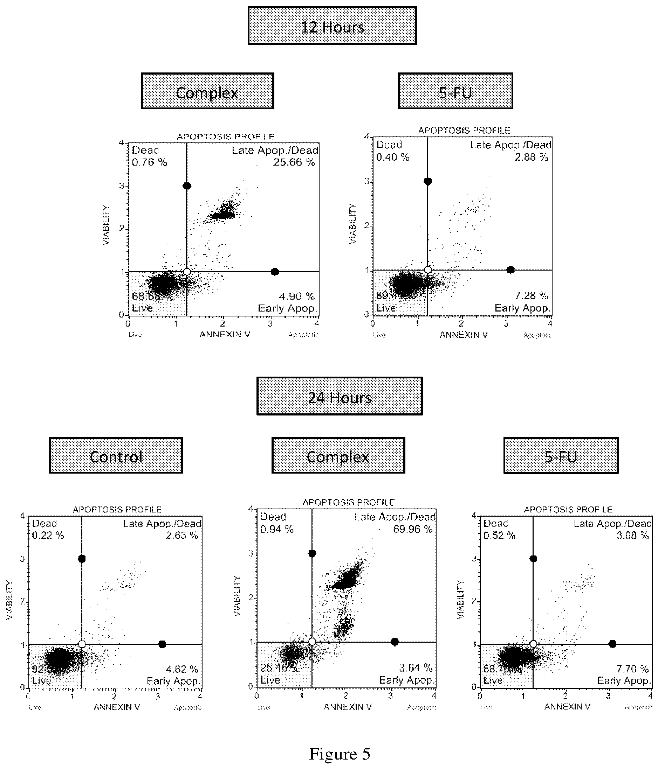

Considering the results shown in Figure 5, in the control group, both Annexin V-FITC and PI are negative, so this proves that control (untreated) group is alive. Considering the 12th hour of [CuCl(5-FU)(terpy)] complex, signals are shown in both Annexin V-FITC and PI positive areas, representing early and late apoptosis. When the 24th hour of the same group is examined, it is stated that apoptosis has increased significantly. Considering the 12th hour results of HT29 cells treated with 5-FU, it has been shown that apoptosis did not increase significantly and this did not further change at the 24th hour.

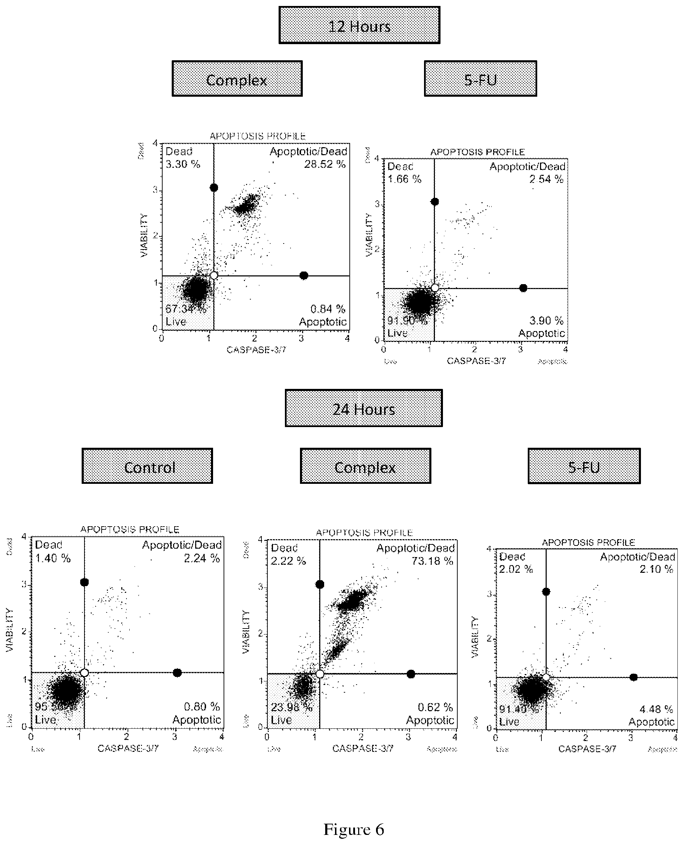

In the present invention, the apoptosis was further confirmed by the Caspase 3/7 test (Figure 6). For the HT29 cell line, 100 µM for [CuCl(5-FU)(terpy)] and 173 µM for 5-FU, which corresponds to the approximate human plasma dose, were used and measurements were performed at 12 and 24 hours.

Caspases (cysteine-aspartic proteases) are a family of protease enzymes that play a key role in apoptosis. They are enzymes specific to apoptosis. They are normally present as zymogens and are activated when the cell undergoes apoptosis. They are responsible for the degradation of various cellular proteins.

Caspase3/7 kit is used in the present invention. The test principle is based on signal reception with a DNA-binding dye that radiates in the presence of activated Caspase 3/7. The 7-Aminoactinomycin D (7-AAD) dye is not permeable to the cell membrane, so it only radiates in dead cells that have impaired membrane integrity.

Considering the results, the control group is both Caspase 3/7 and 7-AAD negative, indicating that the cells are viable. Considering the 12th hour of [CuCl(5-FU)(terpy)] complex, signaling is shown in both Caspase 3/7 and 7-AAD positive areas, representing late apoptosis, and when the 24th hour of the same group is examined, it is stated that caspase activation has increased significantly. Considering the 12th hour results of HT29 cells treated with 5-FU, it has been shown that caspase activation (apoptosis) did not increase significantly and this rate did not change at the 24th hour.

The two apoptosis studies performed (Annexin V-FITC and Caspase 3/7 activation) show parallel results confirming each other.

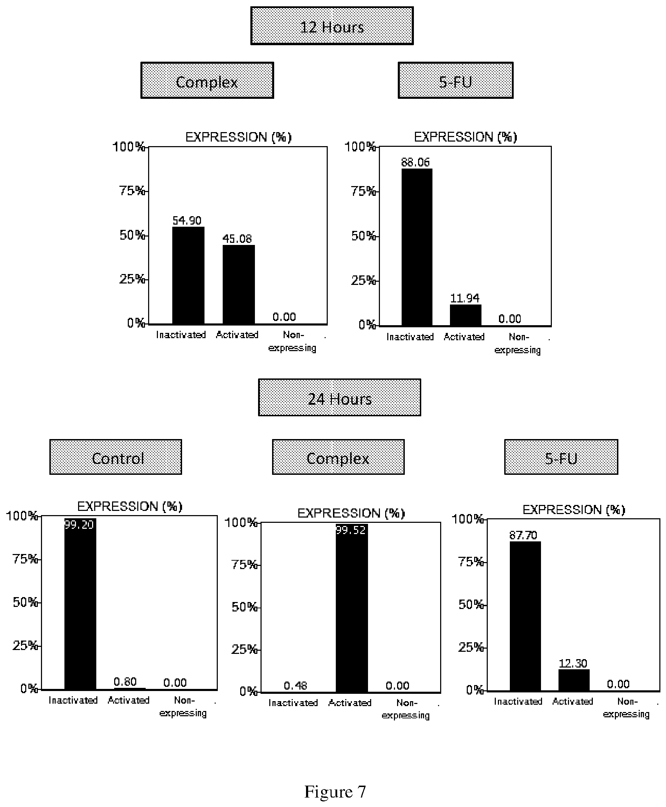

In the present invention, DNA damage rate was investigated in flow cytometry with DNA Damage (SER139) test (Figure 7). For the HT29 cell line, 100 µM for [CuCl(5-FU)(terpy)] and 173 µM for 5-FU, which corresponds to the approximate human plasma dose, were used and measurements were performed at 12 and 24 hours.

DNA damage is a change in the chemical structure of DNA. These are called proteins around which histone DNA is wrapped. Histones are structures that provide strength to the very long DNA chain to hold it together. Histones exist in different types according to their lysine and arginine content. H2A is one of these histone types. H2A also carries different variants within itself and H2A.X is one of them. γH2AX (S139) is the phosphorylated form of H2A.X and is very important since it occurs when DNA double-strand breaks occur. Thus, γH2AX becomes a susceptible target for observing DNA double-strand breaks in cells. Thus, phosphorylation of H2A.X at serine 139 is an important indicator of DNA damage. As the level of DNA damage increases, the level of Phospho Histone H2A.X (also known as γH2AX) accumulates at sites of DNA damage.

The Dual Detection kit is a line of products containing a double antibody; one is used to detect total protein expression (H2A.X) and the other is used to detect the phosphorylated form of the same target (γH2AX (Ser139)).

As shown, H2A.X total protein expression was observed at a rate of 99% in the control group, and γH2AX (S139) expressed as 'activated' was only observed in 0.80%, which means that there was no DNA damage in the control group HT29 cells. Considering the 12th hour of [CuCl(5-FU)(terpy)] complex, γH2AX (Ser139) observed only 45% of DNA damage, while this rate was 99% at 24 hours, which means that DNA damage is observed in almost all cells at 24 hours. Finally, considering the cell group treated with 5-FU, it was shown that there was no significant increase in DNA damage between the 12th hour and the 24th hour.

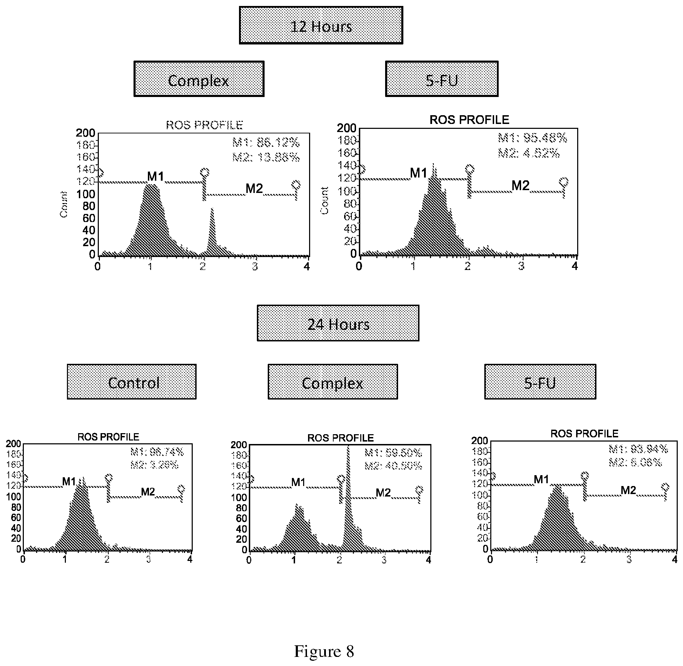

In the present invention, the rate of oxidative stress was investigated in flow cytometry with the Oxidative Stress Kit test (Figure 8). For the HT29 cell line, 100 µM for [CuCl(5-FU)(terpy)] and 173 µM for 5-FU, which corresponds to the approximate human plasma dose, were used and measurements were performed at 12 and 24 hours.

Oxidative stress results from an imbalance between reactive oxygen species and a biological system's ability to detoxify reactive intermediates. Examples of ROS include peroxides, superoxide, hydroxyl radicals.

The Oxidative Stress Kit provides quantitative measurements of cellular populations exposed to oxidative stress based on the detection of Reactive Oxygen Species (ROS), i.e., superoxide. ROS negative and positive cells can be measured in both adherent and suspension.

The Oxidative Stress Kit simultaneously determines the number and percentage of cells exposed to oxidative stress based on the intracellular detection of superoxide radicals. The kit uses Oxidative Stress Reagent for ROS detection in cells. The Oxidative Stress Reagent is based on dihydroetidium (DHE), a well-characterized reagent that is widely used to detect reactive oxidative species in cellular populations. The reactive cell is permeable and it has long been accepted that, after reacting with superoxide anions, DHE undergoes oxidation to combine with the DNA-binding fluorophore ethidium bromide, or DNA, to form a structurally similar product that fluoresces red.

The graphs show two results: ROS (-): viable cells (M1- blue) and ROS (+): Cells displaying ROS (M2- red). According to these results, in the control group, the rate of M1 ROS negative is 96%, while the rate of ROS positive is 4%, and this result shows that the oxidative stress of the control group cells is quite low. Considering the 12th hour of [CuCl(5-FU)(terpy)] complex, while the rate of ROS was positive was 13.88%, this rate was shown to be 40.50% when looked at the 24th hour, thus, it was concluded that [CuCl(5-FU) (terpy)] complex increases oxidative stress in a time-dependent manner. While considering the 12th hour of 5-FU, 4.52% is ROS positive, this rate is expressed as 6.06% when considering the 24th hour, thus, it was concluded that 5-FU did not cause a significant increase in oxidative stress over time compared to [CuCl(5-FU)(terpy)] complex.

Chorioallantoic Membrane (CAM) assay is a study involving the implantation of a material or compound on the extraembryonic membrane of the developing chick egg. Chick embryo develops within 21 days, CAM is formed from day 4, and it grows exponentially until the 14th day, so it has a rapidly developing vascular system. Given these properties, CAM is suitable for angiogenic or antiangiogenic studies.

In the present invention, [CuCl(5-FU)(terpy)] was left on the CAM by applying 50µL on filter papers cut in equal sizes at IC₅₀ = 4.52 µM and GI₅₀ = 1µM doses used for HT29 cells. No treatment was applied to the control group, 50µL of Hank's Buffer Sodium Salt (HBSS) was added on the filter papers in the control group. During Hank's Buffer Sodium Salt (HBSS) CAM studies, [CuCl(5-FU)(terpy)] is used to prepare doses of IC₅₀ = 4.52 µM and GI₅₀ = 1 µM for HT29 cells. The time of treatment is counted as the 0th hour, and then the 24th and 48th hour photos of the same egg are taken and the results are evaluated.

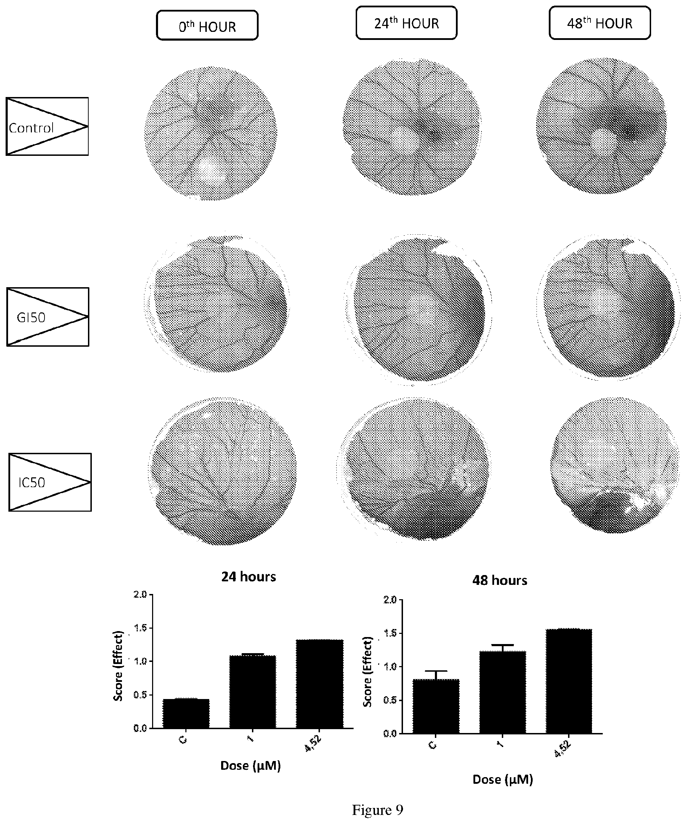

Vascular development and embryo health were prioritized while interpreting the results shown in Figure 9. Considering the control group, an increase in vascular development was observed depending on time and embryo development progressed healthily depending on time. A time-dependent decrease in capillary development was observed in the GI₅₀ group compared to the control group, however, embryo development progressed well. Considering the IC₅₀ group, it was observed that vascular development weakened over time and embryo development became unhealthy and its color was different from normal color depending on time.

All eggs in which the study was carried out were scored according to drug efficacy. Scoring is performed between 0, 0.5, 1 and 2 scores depending on vessel development. Scoring is performed by comparing vessel development over time. While the 24th hour scoring is performed compared to the 0th hour, scoring of 48th hour eggs is compared with 24th hour images.

In expressing the eggs graphically, there is a scoring system and the scoring of the effect. If Score=0 while scoring, it means that the effect is inactive, it means normal embryo development, and it is given to eggs where no change is observed. If the score = 0.5, it means that the effect is weak, there is normal embryo development, and in addition thereto, weak effects are observed in the blood vessels. If the score = 1, it means that the effect is strong, normal embryo development can be observed, and the growth of blood vessels is marginally reduced. If the score=2, it means that the effect is very strong, normal embryo development may or may not be observed, and it is the score in which significant weakening of the vessels around the disc/filter paper is observed.

When the graphs in Figure 9 are examined, it is shown that the Control group is the group with the lowest score as it should be. There was an increase in the scores of the three groups depending on time. When both time frame graphs were examined, it was stated that the group with the highest score, that is, the effect, was the eggs applied IC₅₀. While the GI₅₀ was close to score = 1 at the 24th hour, it was observed that this ratio was between score = 1 and score = 1.5 at the 48th hour.

## Claims

1. Copper (II) complex containing 5-fluorouracil and terpyridine ligand, having the formula [CuCl(5-Fu)(terpy)].

2. A complex according to Claim 1 for use in cancer treatment.

3. A complex according to Claim 2 for use in the treatment of lung cancer, breast cancer, or prostate cancer.

4. A complex according to Claim 2 for use in the treatment of colon cancer.

5. Synthesis method of the complex according to Claim 1, including following steps.
a. Preparing an aqueous solution of Na(5-FU)·H₂O (0,50 mmol) in 5 mL water,
b. Preparing an aqueous solution of CuCl₂ (0.25 mmol) in 5 mL water,
c. Preparing a solution of terpy (0.25 mmol) in 10 mL methanol,
d. Adding the solution in step "a" to the solution prepared in step "b" with stirring,
e. Adding the solution in step "c" into the mixture,
f. Stirring and filtering the solution at 50-65 °C for 2-4 hours,
g. Filtering and air drying the crystals of the complex.

## Patentansprüche

1. Kupfer(II)-Komplex mit 5-Fluoruracil und Terpyridin-Liganden, Formel [CuCl(5-Fu)(terpy)].

2. Ein Komplex nach Anspruch 1 zur Anwendung in der Krebsbehandlung.

3. Ein Komplex nach Anspruch 2 zur Anwendung bei der Behandlung von Lungenkrebs, Brustkrebs oder Prostatakrebs.

4. Ein Komplex nach Anspruch 2 zur Anwendung bei der Behandlung von Dickdarmkrebs.

5. Syntheseverfahren für den Komplex nach Anspruch 1, umfassend die folgenden Schritte
a. Herstellung einer wässrigen Lösung von Na(5-FU)·H₂O (0,50 mmol) in 5 mL Wasser,
b. Herstellung einer wässrigen Lösung von CuCl₂ (0.25 mmol) in 5 mL Wasser,
c. Herstellung einer Lösung von Terpy (0.25 mmol) in 10 mL Methanol,
d. Die Lösung aus Schritt "a" wird unter Rühren zu der in Schritt "b" hergestellten Lösung gegeben,
e. Die Lösung aus Schritt "c" wird der Mischung hinzugefügt,
f. Die Lösung wird für 2-4 Stunden bei 50-65 °C gerührt und filtriert,
g. Filtration und Lufttrocknung der Kristalle des Komplexes.

## Revendications

1. Complexe de cuivre (II) contenant du 5-fluorouracile et un ligand terpyridine, ayant la formule [CuCl(5-Fu)(terpy)].

2. Complexe selon la revendication 1 pour une utilisation dans le traitement du cancer.

3. Complexe selon la revendication 2 pour une utilisation dans le traitement du cancer du poumon, du cancer du sein ou du cancer de la prostate.

4. Complexe selon la revendication 2 pour une utilisation dans le traitement du cancer du côlon.

5. Procédé de synthèse du complexe selon la revendication 1, comprenant les étapes suivantes :
a. Préparer une solution aqueuse de Na(5-FU)•H₂O (0.50 mmol) dans 5 mL d'eau,
b. Préparer une solution aqueuse de CuCl₂ (0.25 mmol) dans 5 mL d'eau,
c. Préparer une solution de terpy (0.25 mmol) dans 10 mL de méthanol,
d. Ajouter la solution de l'étape « a » à la solution préparée à l'étape « b » sous agitation,
e. Ajouter la solution de l'étape « c » au mélange,
f. Agiter et filtrer la solution à 50-65 °C pendant 2-4 heures,
g. Filtrer et sécher à l'air les cristaux du complexe.
